Europäisches Patentamt

European Patent Office

Office européen des brevets

(11) Veröffentlichungsnummer: **0 369 044**
**A1**

(12) **EUROPÄISCHE PATENTANMELDUNG**

(21) Anmeldenummer: 88118987.2

(51) Int. Cl.⁵: **A61N 1/05**

(22) Anmeldetag: 14.11.88

(43) Veröffentlichungstag der Anmeldung:
23.05.90 Patentblatt 90/21

(84) Benannte Vertragsstaaten:
CH DE FR GB IT LI NL SE

(71) Anmelder: **Siemens Elema AB**
**Röntgenvägen 2**
**S-171 95 Solna 1(SE)**

(84) **SE**

Anmelder: **Siemens Aktiengesellschaft**
**Wittelsbacherplatz 2**
**D-8000 München 2(DE)**

(84) **CH DE FR GB IT LI NL**

(72) Erfinder: **Hirschberg, Jakub, Dipl-Ing.**
**Aprilvägen 3**
**S-183 44 Täby(SE)**

(54) **Elektrodenanordnung.**

(57) Elektrodenanordnung zur Stimulation von Körpergewebe, insbesondere für die intrakardiale Stimulation des Herzens, mit mindestens einem langgestreckten Leiter, der von einer elektrischen Isolierung umgeben ist. Damit die Elektrodenleitung der Elektrodenanordnung dünn ist und eine ausreichende Elastizität und Schmiegsamkeit aufweist gleichzeitig damit, dass sie eine hohe Zugfestigkeit erhält, wird erfindungsgemäss vorgeschlagen, dass der Elektrodenleiter (5,10) aus einem Flechtschlauch (9,14, 15) besteht.

# FIG 1

EP 0 369 044 A1

# Elektrodenanordnung

Die Erfindung betrifft eine Elektrodenanordnung zur Stimulation von Körpergewebe, insbesondere für die intrakardiale Stimulation des Herzens, mit mindestens einem langgestreckten elektrischen Leiter, der von einer elektrischen Isolierung umgeben ist.

Bei den heutigen Elektrodenanordnungen, z.B. bei Herzschrittmacherelektroden, ist es erforderlich, dass die Elektrodenleitung dünn und biegsam ist, damit eine insgesamt elastische und schmiegsame Elektrode erhalten wird. Die herkömmlichen langgestreckten Leiter einer Elektrodenleitung sind daher wendelförmig gewickelt. Wenn bei einer Herzschrittmacherimplantation die Lage der im Herzen applizierten Elektrode geändert werden soll, muss an dem proximalen Ende der Herzschrittmacherelektrode gezogen werden. Bei einem grossen Widerstand der Elektrode, z.B. wenn der Elektrodenkopf im Trabekelwerk verhakt ist, kann die Elektrodenleitung beim Ziehen bleibend verformt werden, wobei die Wendeln der Leiter auseinandergehen. Um dies zu vermeiden, kann z.B. die Isolierung entsprechend dick gemacht werden, was aber den Durchmesser der Elektrodenleitung in unerwünschter Weise vergrössert.

Wenn bei einer bipolaren Herzschrittmacherelektrode als indifferente Elektrode eine Ringelektrode auf dem Leiter angebracht ist, wird dieser Teil der Elektrode steif. Um Stromverluste zu vermeiden, muss trotzdem die Oberfläche der Ringelektrode verhältnismässig gross sein, so dass in diesem Bereich die Stromdichte niedrig gehalten werden kann. Um zu verhindern, dass die Ringelektrode lang und dadurch steif gemacht wird, wurde bisher stattdessen mehrere kleinere Ringelektroden nacheinander auf der Elektrodenleitung appliziert, was den Aufbau der Herzschrittmacherelektrode erschwert.

Der Erfindung liegt die Aufgabe zugrunde, eine Elektrodenanordnung der eingangs genannten Art zu schaffen, deren Elektrodenleitung dünn ist und eine ausreichende Elastizität und Schmiegsamkeit aufweist gleichzeitig damit, dass sie eine hohe Zugfestigkeit erhält. Eine weitere Aufgabe der Erfindung ist es, die Elastizität im Bereich der indifferenten Elektrode beizubehalten, ohne die elektrischen Eigenschaften der Elektrode zu verschlechtern.

Diese Aufgabe ist erfindungsgemäss dadurch gelöst, dass mindestens ein Leiter aus einem Flechtschlauch besteht. Die einzelnen Drähte des als Flechtschlauch ausgebildeten Leiters können sehr dünn gehalten werden, was insgesamt eine hohe Flexibilität der Elektrodenleitung ergibt. Die gesamte Elektrodenleitung kann hierdurch auch dünn gehalten werden. Durch die sich kreuzenden Drähte des Flechtschlauches, die sich gegenseitig abstützen, ist eine ausserordentlich hohe Zugfestigkeit des Leiters bzw. der Elektrodenleitung gegeben. Die sich kreuzenden Drähte bilden Parallelogramme, deren Verformungsmöglichkeiten die Dehnbarkeit des Flechtschlauches begrenzen. Durch diesen Aufbau wird ferner erreicht, dass die Drähte nach einer Zugbelastung des Flechtschlauches in ihre Ausgangslage zurückgehen.

In einer vorteilhaften Ausbildung der Erfindung wird vorgeschlagen, dass der Flechtschlauch aus Drähten und Kunststoffäden derart geflochten ist, dass die Drähte durch die Kunststoffäden elektrisch gegeneinander isoliert sind. Ein solcher Flechtschlauch kann daher für eine multipolare Herzschrittmacherelektrode vorgesehen sein.

Eine weitere Ausgestaltung der Erfindung ergibt sich dadurch, dass die Elektrodenanordnung einen langgestreckten Kern aufweist und der Flechtschlauch über mindestens einen Teilabschnitt davon verläuft. Dadurch ist erreicht, dass sich der Flechtschlauch beim Ziehen an der Elektrodenleitung zusammenzieht und dicht um den Kern legt, so dass dieser in diesem Bereich nicht ausgezogen bzw. verformt wird. Dieser Effekt kann mit besonderem Vorteil für Elektrodenleitungen ausgenutzt werden, deren Kern aus einem wendelförmigen elektrischem Leiter besteht. Im Gegensatz zu den herkömmlichen wendelförmigen Leitern wird hierdurch vermieden, dass die Wendel bei Zugbelastung unerwünscht elastisch oder plastisch gedehnt wird.

Eine besonders günstige Ausgestaltung wird erhalten, indem mindestens ein Teil des Flechtschlauches von der elektrischen Isolierung freigelegt ist. Der freigelegte Bereich des Flechtschlauches kann bei z.B. einer bipolaren Herzschrittmacherelektrode als indifferente Elektrode dienen. Dabei ergibt sich der Vorteil, dass die indifferente Elektrode beliebig gross gehalten werden kann gleichzeitig damit, dass sie genauso elastisch und schmiegsam ist wie die übrige Elektrodenleitung, ohne dabei die elektrischen Eigenschaften der Elektrode zu verschlechtern. Bei einer multipolaren Herzschrittmacherelektrode können mehrere koaxial liegende Flechtschläuche vorgesehen sein, bei der für jeden Flechtschlauch jeweils ein Bereich des Flechtschlauches von der Isolierung freigelegt wird.

Im Hinblick auf eine weitere Ausgestaltung der Erfindung wird vorgeschlagen, dass der Flechtschlauch multifilar aufgebaut ist. Auch dieser Flechtschlauch kann wegen der einzeln isolierten Drähte mit Vorteil bei einer multipolaren Herzschrittmacherelektrode verwendet werden.

Im Bereich der Hochfrequenzübertragung werden zwar Koaxialkabel verwendet, die geflochtene Schläuche aufweisen, die aber lediglich als Abschirmung gegen äussere Störsignale dienen. Derartigen Kabeln wird nicht die Bedeutung hinsichtlich Flexibilität und Schmiegsamkeit beigemessen, wie es bei z.B. Herzschrittmacherelektrodenleitungen der Fall ist. Die Verhinderung einer Verformung eines inneren Leiters ist dabei ebenfalls nicht vorgesehen.

Die Erfindung ist nachfolgend anhand von mehreren, in den Zeichnungen dargestellten Ausführungsbeispielen näher erläutert. Es zeigen:

FIG 1 eine Seitenansicht einer Herzschrittmacherelektrode nach der Erfindung und

FIG 2 u. 3 Seitenansichten weiterer Ausführungsformen einer Herzschrittmacherelektrode nach der Erfindung.

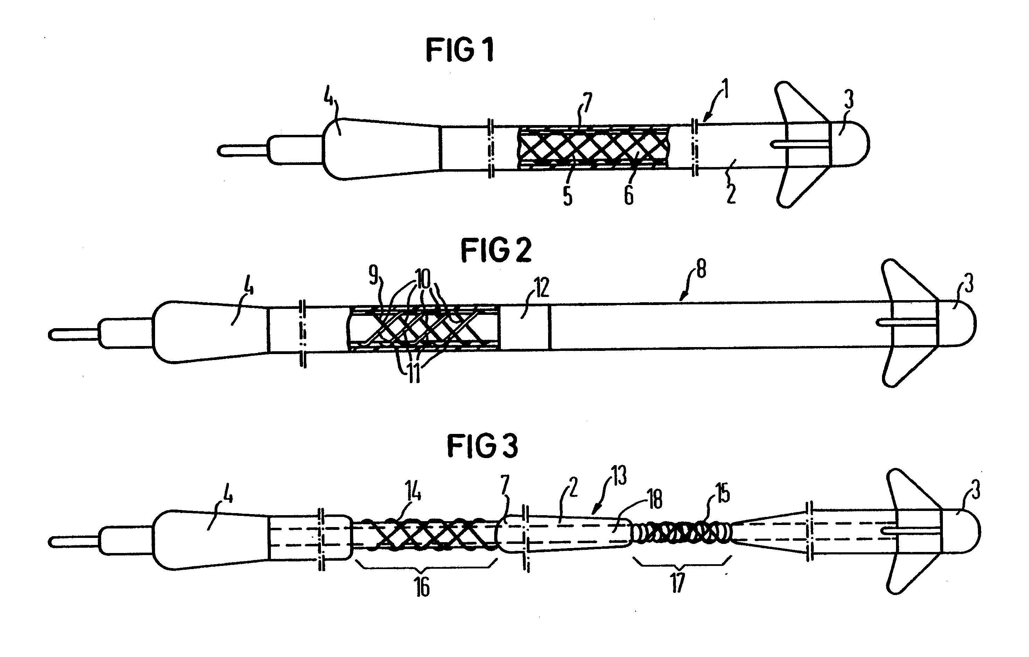

In der FIG 1 ist eine unipolare Herzschrittmacherelektrode (1) mit einer Elektrodenleitung (2), einem Elektrodenkopf (3) und einem Elektrodenanschlussteil (4) dargestellt. Die Elektrodenleitung (2) weist einen langgestreckten elektrischen Leiter (5) auf, der um einen Kern (6) aus z.B. einem Kunststoffschlauch und entlang desselben verläuft. Aus dem Teilschnitt ist ersichtlich, dass der Leiter (5) aus einem Flechtschlauch besteht, der mit einer elektrischen Isolierung (7) versehen ist.

FIG 2 zeigt eine bipolare Herzschrittmacherelektrode (8) mit einem als Leiter dienenden Flechtschlauch (9), der aus Drähten (10) und Kunststoffäden (11) geflochten ist. Die parallel verlaufenden elektrisch leitenden Drähte (10) sind dadurch gegeneinander isoliert. Die Kunststoffäden (11) dienen hier als Abstandshalter. In diesem Ausführungsbeispiel kann der eine Leiter zu einem auf der Elektrodenleitung (2) angebrachten indifferenten Elektrodenring (12) und der weitere Leiter zum Elektrodenkopf (3) führen.

Die Drähte (10) können auch einzeln isoliert sein. Wenn statt Kunststoffäden (11) einzeln isolierte Drähte vorgesehen sind, kann dieser Flechtschlauch (9) als ein multifilarer Leiter dienen.

In der FIG 3 ist eine multipolare Herzschrittmacherelektrode (13) mit zwei koaxial verlaufenden, als elektrische Leiter vorgesehenen Flechtschläuchen (14,15) dargestellt. In dieser FIG ist gezeigt, dass jeweils ein Teil eines Flechtschlauches (14,15) von der elektrischen Isolierung (7) freigelegt ist. Die freigelegten Teile (16,17) der Flechtschläuche (14,15) dienen nun als separate Elektroden. Diese Elektrodenabschnitte sind mindestens ebenso flexibel wie der übrige Elektrodenleiter.

In dieser FIG 3 ist ferner gezeigt, dass ein weiterer, durch die gesamte Elektrodenleitung verlaufender wendelförmiger Leiter (18) angeordnet ist. Der Leiter (18) ist vorzugsweise isoliert. Der Flechtschlauch (15) kann z.B. über die gesamte Länge der Elektrodenleitung (2) bis zum Elektrodenkopf (3) verlaufen. Hierdurch erhält der wendelförmige Leiter (18) einen derartigen Halt, dass er beim Ziehen an der Herzschrittmacherelektrode (13) nicht verformt wird.

Die Elektrodenanordnung nach der Erfindung kann z.B. auch als Defibrillatorelektrode oder Nervenstimulierungselektrode vorgesehen sein.

Bezugszeichenliste

1,8,13 Herzschrittmacherelektrode
2 Elektrodenleitung
3 Elektrodenkopf
4 Elektrodenanschlussteil
5,18,10 Leiter, Drähte
6 Kern
7 Isolierung
9,14,15 Flechtschlauch
11 Kunststofäden
12 Elektrodenring
16,17 Teile

**Ansprüche**

1. Elektrodenanordnung zur Stimulation von Körpergewebe, insbesondere für die intrakardiale Stimualtion des Herzens, mit mindestens einem langgestreckten Leiter, der von einer elektrischen Isolierung umgeben ist, **dadurch gekennzeichnet,** dass der Leiter (5,10) aus einem Flechtschlauch (9,14,15) besteht.

2. Elektrodenanordnung nach Anspruch 1, **dadurch gekennzeichnet,** dass mindestens zwei koaxial zueinander angeordnete Flechtschläuche (14,15) vorgesehen sind.

3. Elektrodenanordnung nach Anspruch 1 oder 2, **dadurch gekennzeichnet,** dass der Flechtschlauch (9,14,15) derart aus Drähten (10) und Kunststoffäden (11) geflochten ist, dass die Drähte durch die Kunststoffäden elektrisch gegeneinander isoliert sind.

4. Elektrodenanordnung nach einem der Ansprüche 1 bis 3, **dadurch gekennzeichnet,** dass diese einen langgestreckten Kern (6) aufweist und der Flechtschlauch (9,14,15) über mindestens einen Teilabschnitt davon verläuft.

5. Elektrodenanordnung nach Anspruch 4, **dadurch gekennzeichnet,** dass der Kern (6) ein wendelförmiger elektrischer Leiter (18) ist.

6. Elektrodenanordnung nach einem der Ansprüche 1 bis 5, **dadurch gekennzeichnet,** dass mindestens ein Teil (16,17) des Flechtschlauches (14,15) von der elektrischen Isolierung freigelegt ist.

7. Elektrodenanordnung nach einem der Ansprüche 1 bis 6, **dadurch gekennzeichnet,** dass der Flechtschlauch (9,14,15) multifilar aufgebaut ist.

FIG 1

FIG 2

FIG 3

EP 0 369 044 A1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | FR-A-2 446 001 (CARDIOFRANCE) <br> * Seite 3, Zeile 1 - Seite 5, Zeile 14; Seite 5, Zeile 29 - Seite 6, Zeile 9; Figuren 1,2,5 * <br> --- | 1,2,4-6 | A 61 N 1/05 |
| X | US-A-4 295 270 (CAMMARATA) <br> * Spalte 2, Zeilen 57-68; Figuren 1,2 * | 1 | |
| A | <br> --- | 4,6 | |
| X | EP-A-0 249 338 (C.R. BARD) <br> * Spalte 8, Zeilen 5-51; Spalte 10, Zeile 5 - Spalte 11, Zeilen 8,37-51; Figuren 1,5-9 * | 1,7 | |
| A | <br> --- | 3 | |
| X | DE-A-3 020 588 (BISPING) <br> * Ansprüche 1-3,6,7,9,11; Figur 1 * | 1,2,4-6 | |
| A | <br> --- | 3 | |
| X | US-A-3 760 812 (TIMM) <br> * Insgesamt * <br> ----- | 1,3,4,7 | |

**RECHERCHIERTE SACHGEBIETE (Int. Cl.4)**

A 61 N

Der vorliegende Recherchenbericht wurde für alle Patentansprüche erstellt

| Recherchenort | Abschlußdatum der Recherche | Prüfer |
|---|---|---|
| DEN HAAG | 06-06-1989 | SCHMIERER U.J. |